# EUROPÄISCHE PATENTSCHRIFT

(11) **EP 0 966 262 B1**
(45) Veröffentlichungstag und Bekanntmachung des Hinweises auf die Patenterteilung: **17.04.2002**
(21) Anmeldenummer: 98905365.7
(22) Anmeldetag: 29.01.1998
(51) Int. Cl.: A61K 7/48

(54) **HAUTPFLEGEMITTEL**
SKIN CARE PRODUCT
AGENTS DE SOIN POUR LA PEAU

(30) Priorität: 07.02.1997 DE 19704693
(43) Veröffentlichungstag der Anmeldung: 29.12.1999
(73) Patentinhaber: Henkel Kommanditgesellschaft auf Aktien, 40589 Düsseldorf (DE)
(72) Erfinder: WALDMANN-LAUE, Marianne, D-40789 Monheim (DE); BORDAT, Pascal, D-40699 Erkrath (DE); MUNK, Gabriele, D-40477 Düsseldorf (DE); JASSOY, Claudia, D-40235 Düsseldorf (DE); HÖRNER, Viola, D-40593 Düsseldorf (DE)
(86) Internationale Anmeldenummer: EP9800473
(87) Internationale Veröffentlichungsnummer: WO9834589

(56) Entgegenhaltungen:
- EP-A- 0 103 878
- FR-A- 2 652 742

## Beschreibung

Die Erfindung betrifft kosmetische und dermatologische Zubereitungen zur Pflege empfindlicher Haut in Form einer Emulsion oder Creme, die aufgrund besonders ausgewählter Fettkomponenten, Emulgatoren und Wirkstoffe nicht nur eine geringe Hautreizung aufweisen, sondern auch die Vitalität des Hautgewebes steigern.

Die kosmetische Pflege der empfindlichen Haut gewinnt immer mehr an Bedeutung, da heute mehr als die Hälfte der Verbraucherinnen ihre Haut als empfindlich einstufen. Der Begriff der empfindlichen Haut ist in der kosmetischen Literatur nicht eindeutig definiert. es sind aber Testmethoden bekannt geworden, die es erlauben, das Reizpotential von Hautbehandlungsmitteln recht genau zu prüfen und Aussagen über positive Wirkungen, z.B. über die Wirkung auf den Zellstoffwechsel der Haut, zu machen.

Polysaccharide, Zucker und Glycoproteine sind in der Kosmetik als hautfeuchthaltende Komponenten bekannt. Aus FR-A-2 652 742 waren auch schon die antiallergischen Wirkungen eines Zusatzes von L-α-Fucose, L-Rhamnose und L-Xylose zu kosmetischen und dermatologischen Zusammensetzungen bekannt.

Es hat sich aber herausgestellt, daß eine erhebliche Verbesserung der Hautwirkung solcher Zubereitungen erreicht werden kann, wenn auch die Trägerkomponenten, im Falle von Emulsionen und Cremes, also die Öl- und Fettkomponenten und Emulgatoren, bezüglich ihres Reizpotentials weiter optimiert werden.

Gegenstand der Erfindung sind daher verbesserte Zusammensetzungen zur Pflege empfindlicher Haut in Form einer Emulsion oder Creme, die als Öl- oder Fettkomponenten natürliche Öle- und Fette oder Wachse oder Ester linearer Fettsäuren mit 8 bis 22 C-Atomen und als Emulgatoren Phospholipide, Sterine, Alkyl-(oligo)-glycoside, Fettsäureester von Zuckern, Zuckeralkoholen oder Polyglycerin oder Gemische davon und als Wirkstoffe Desoxyzucker oder diese enthaltende Naturstoffe enthalten.

Als Desoxyzucker im Sinne der Erfindung werden vorzugsweise die L(-)-Fucose und die L(+)-Rhamnose verstanden. Es eignen sich aber auch Naturstoffe, die solche Desoxy-Zuckerbausteine enthalten, z.B. Polysaccharide oder Pflanzenglycoside. Die Fucose kommt z.B. als Polysaccharid-Baustein in einem aus der Meeres-Braunalge (fucus vesiculosus) isolierten Polysaccharid-Gel vor, Rhamnose stellt einen Polysaccharid-Baustein der Arabinsäure in Gummi arabicum dar. Bevorzugt sind Fucose, Rhamnose oder ein Gemisch davon in Mengen von 0,1 - 10 Gew.-% in der Zusammensetzung enthalten.

Als Träger dieser Wirkstoffe eignen sich Emulsionen bzw. Cremes, die geeignet sind, die Wirkstoffe in feiner Verteilung und ohne hautreizende Nebenwirkungen auf die Haut aufzubringen. Es hat sich überraschend gezeigt, daß übliche Kohlenwasserstoffe, z.B. Paraffinöle, Vaseline (Petrolatum) oder Silikone für diesen Zweck weniger gut geeignet sind und daher in den erfindungsgemäßen Zubereitungen nur in untergeordneten Mengen enthalten sein sollen.

Überwiegend sollte die Öl- oder Fettphase vielmehr aus natürlichen Ölen, Fetten und Wachsen oder aus Estern linearer Fettsäuren mit 8 bis 22 C-Atomen bestehen. Solche geeigneten Öle bzw. Fette sind z.B. Olivenöl, Sonnenblumenöl, raffiniertes Sojaöl, Palmöl, Rapsöl, Sesamöl, Mandelöl, Boretschöl, Nachtkerzenöl, Jojobaöl, Kokosöl, Sheabutter, Spermöl, Klauenöl, Rindertalg und Schweineschmalz. Weiterhin eignen sich Ester linearer Fettsäuren mit 8 - 22 C-Atomen, z.B. Oleyloleat, Oleylerucat. Hexyllaurat, Capryl- und Caprinsäure-Triglycerid, Isopropylplamitat oder Butylstearat. Bevorzugt sind Öle mit einem hohen Anteil an Gammalinolensäure (6,9,12-Octadecatfiencarbonsäure) in einer Menge von 10 - 50 Gew.-% der Ölphase enthalten.

Besonders bevorzugt enthalten die erfindungsgemäßen Zusammensetzungen als Öl- oder Fertkomponente Sheabutter (Karite-Fett) aus dem Samen der Pflanze Butyrospermum Parkii. Mandelöl. Nachtkerzenöl oder ein Capryl- und Caprinsäure-Triglycerid oder Mischungen aus diesen Fettstoffen in einer Menge von 10 - 30 Gew.-% der Zusammensetzung. Hingegen sollte die Öl- oder Fettphase nicht mehr als 5 Gew.-% an Kohlenwasserstoffen oder Silikonölen enthalten.

Als Emulgatoren geeignete Phospholipide sind vor allem die Glucose-Phospholipide, die z.B. als Lecithine bzw. Phosphatidylcholine aus z.B. Eidotter oder Pflanzensamen (z.B. Sojabohnen) gewonnen werden.

Als Sterine wird eine Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.

Alkyl-(oligo)-glycoside sind Verbindungen der allgemeinen Formel RO(G)ₓ, in der R eine lineare Alkylgruppe mit 8 bis 18 C-Atome, G ein Glycosidrest, z.B. der von Glucose abgeleitete Glucosidrest oder ein z.B. von Mannose oder Fructose abgeleiteter Mannosid- oder Fructosidrest ist und x dessen Oligomeriationsgrad bevorzugt einen Wert zwischen 1 und 2 hat. Solche Alkylglycoside sind im Handel z.B. unter dem Warenzeichen Plantaren® oder Plantacare® erhältlich. Gemische aus Alkyl-(oligo)-glucosiden und Fettalkoholen sind zum Beispiel unter der Bezeichnung Montanov® 68 oder Emulgade® PL 68/50 im Handel.

Fettsäureester von Zuckern, Zuckeralkoholen wie Sorbit und von Polyglycerinen sind ebenfalls bekannte und im Handel erhältliche Emulgatoren.

Bevorzugt enthalten die erfindungsgemäßen Zusammensetzungen keine oder nicht mehr als 1 Gew-% an Sulfat- oder Sulfonattensiden. Ethylenoxid- oder Propylenoxid-Addukten und quartären Ammoniumtensiden.

Neben den Öl- und Fettkomponenten und den Emulgatoren kann die Öl- und Fettphase noch weitere Lipidstoffe enthalten, die der Stabilisierung der Zusammensetzungen oder der Pflege der Haut dienen. Solche geeigneten Lipidstoffe sind z.B. Tocopherole (Vitamin E) oder Ceramide. Unter Ceramiden werden N-Acylsphingosin (Fettsäureamide des Sphingosins) oder synthetische Analogen solcher Lipide (sogenannte Pseudo-Ceramide) verstanden, die das Wasserhaltevermögen des Straturn corneum deutlich verbessern.

Schließlich können die erfindungsgemäße Zusammensetzungen alle bekannten und in solchen Zubereitungen üblichen Hilfs- und Zusatzstoffe enthalten, die zur Verbesserung der ästhetischen. anwendungstechnischen und kosmetischen Eigenschaften bekannt sind. Solche Stoffe sind z.B. natürliche oder synthetische Hydrocolloide, z.B. Cellulosederivate, Pflanzengumme, Gelatine, Biopolymere oder auch synthetische, wasserlösliche Polymere zur Verdickung der wäßrigen Phase, Schichtsilikate, Seifen, z.B. Calcium, Magnesium- oder Zinkseifen zur Verdickung der Ölphase, Duftstoffe, Farbstoffe, Pigmente, Konservierungsmittel, Komplexbildner, Polyole wie z.B. Propylenglycol, Dipropylenglycol, Sorbit oder Glycerin und kosmetische Wirkstoffe wie z.B. Allantoin, Bisabolol, Extrakte aus pflanzlichem, tierischem Gewebe oder aus Mikroorganismen (z.B. Hefeextrakte), Vitamine oder Proteine.

Bevorzugt ist in der wäßrigen Phase ein natürliches oder synthetisches Hydrocolloid in einer Menge von 0,01 bis 5 Gew.-% der Zusammensetzung enthalten.

Die Herstellung der erfindungsgemäßen kosmetischen Zusammensetzungen erfolgt in der üblichen Weise, indem man die lipidlöslichen Stoffe mit der Öl- oder Fettphase bis zur klaren Schmelze erwärmt und homogenisiert und die wasserlöslichen Bestandteile in dem Wasser löst und die erwärmte wäßrige Phase unter Rühren in die Fettphase einemulgiert. Die Emulgierung kann unter Zuhilfenahme von Homogenisatoren erfolgen. Die Einarbeitung der Desoxyzucker erfolgt entweder in die Ölphase oder in die fertige Emulsion. Auch Duftstoffe und temperaturempfindliche Wirkstoffe oder Pflanzenextrakte werden bevorzugt nachträglich in die Emulsion eingearbeitet.

Die nachfolgenden Beispiele sollen den Gegenstand der Erfindung näher erläutern:

### Beispiele

### 1.1 Prüfung der Hautverträglichkeit mit dem wiederholten, prolongierten Patch Test (DAPPT)

Die Prüfmethode und die Auswertung der Ergebnisse erfolgt in Anlehnung an den literaturbekannten Duhring Chamber Test (vgl. I. Tausch et al, Parfümerie und Kosmetik, 76. (1/96) S. 28 - 31). Zur deutlicheren Differenzierung gut verträglicher Produkte wurden aber im DAPPT die mit Hilfe von Aluminiumkammern auf dem Rücken von 20 Probanden fixierten Proben über eine erste Expositionszeit von 48 Stunden und eine zweite unmittelbar anschließende Expositionszeit von 72 Stunden getestet. Danach wurde über einen Zeitraum von weiteren 72 Stunden die durch die Proben verursachte Veränderung der Haut visuell bewertet. Es wurde zwischen Erythem, Schuppung, Ödem und Fissur unterschieden.

Aus den Daten aller Probanden über alle Ablesezeitpunkte wurden sowohl für jeden Parameter einzeln als auch für die Summe der Parameter (Reizsummen-Score) Mittelwerte gebildet.

### 1.2 Geprüfte Hautpflegemittel-Rezepturen

**Tabelle I**

| **Bestandteile** | **1V** | **2V** | **3** | **4** | **5** |
|---|---|---|---|---|---|
| Lipoid S-75 | 3,0 | - | - | 3,0 | - |
| Montanov® 68 | - | 5,0 | 5,0 | - | - |
| Generol® 122N | - | 0,5 | 0,5 | - | 1,0 |
| Ceramide HO₃ | - | 0,2 | 0,2 | - | - |
| Bienenwachs | 1,5 | - | - | 1,5 | - |
| Cetiol SB 45 | 9,0 | 1,0 | 1,0 | 9,0 | 3,0 |
| Cetiol J 600 | 5,0 | - | - | 5,0 | - |
| Myritol. 318 | - | 4,0 | 4,0 | - | - |
| Cetiol SN | - | 5,0 | 5,0 | - | - |
| Mandelöl | 5,0 | 2,0 | 2,0 | 5,0 | - |
| Nachtkerzenöl | - | 5,0 | 5,0 | - | 5,0 |
| Rhamnose | - | - | 1,5 | 1,5 | 1,5 |
| Fucogel 1000 | - | - | 2,5 | 2,5 | 2,5 |
| Tufskin | - | - | 1,5 | 1,5 | 1,5 |
| Bisabolol | - | - | 0,1 | 0,1 | 0,1 |
| Allantion | - | - | 0.2 | 0,2 | 0,2 |
| Glycerin | 5,0 | - | - | 5,0 | - |
| Dipropylenglycol | - | 5,0 | 5,0 | - | - |
| Carbopol 980 | - | 0,005 | 0,005 | - | - |
| Carragheenan | 2,0 | - | - | 2,0 | - |
| Wasser, Konservierungsmittel | ad 100 | ad 100 | ad 100 | ad 100 | |
| Verträglichkeitsparamter | | | | | |
| Erythemscore | 0 | 0,17 | 0,06 | 0 | n.b. |
| Summenreizscore | 0,11 | 0,44 | 0,06 | 0 | n.b. |

Bei weniger verträglichen Hautcremes, die im Test einen Erythemscore von 1 bis 2 und einen Summenreizscore 4 bis 5 zeigten, konnte die Verträglichkeit durch Zusatz eines Wirkstoffkomplexes gemäß Rezeptur 5 (in der der Rest zu 100 Gew.-% aus der Vergleichscreme besteht) hinsichtlich der Reizscores um 85 % verbessert werden.

Es wurden die folgenden Handelsprodukte verwendet. (Zur Erläuterung wird die INCI-Nomenklatur verwendet, wo dies möglich ist.)
- Lipoid S-75 :: Hydrogenated Lecithin
- Montanov 68 :: Cetearyl Glucoside. Cetearylalkohol
- Generol 122N:: Soy Sterol
- Ceramide HO₃:: Trihydroxy-Palmitamidohydroxypropyl-Myristyl-Ether
- Cetiol SB 45 :: Butyrospermum Parkii (Sheabutter)
- Cetiol J 600 :: Oleyl Erucate
- Myritol 318 :: Capryl/Capric Triglycerides
- Cetiol SN :: Cetearyl Isononamoate
- Tufskin :: Sorbitol und Yeast Extract
- Fucogel 1000 :: Biosaccharide Gum (reich an Fucose)
- Carbopol 980 :: Carbomer (Polyacrylsäure, vernetzt).

### 2. Prüfung der Vitalitätssteigerung am Humanhautmodell

Für diese Untersuchungen wurde ein dreidimensionales Hautmodell verwendet, welches aus einer Kultur von Humanhautzellen besteht (Skin² von Advanced Tissue Sciences, La Jolla, CA, USA, vgl. Alternative Methods in Toxicology, Vol. 10, B III-4, Seiten 121 - 131).

Die zu prüfenden Cremes wurden auf ein Filterpapier aufgetragen, dieses dann für je 2 Stunden mit der gecremten Seite auf die Oberfläche der Hautkultur gelegt, so daß sie einwirken konnten.

Die Vitalität der Hautkulturen wurde anhand des Vitalfarbstoffs Alamarblau überprüft. Lebende Zellen reduzieren das Farbstoffmolekül, dabei kommt es zu einem Farbstoffumschlag, dessen Intensität photometrisch gemessen wurde. Je vitaler die Kulturen sind, desto höher ist die Reduktionsrate. Die Stoffwechselaktivität wurde über die Messung des Glucoseverbrauchs ermittelt. Glucose ist der Hauptnährstoff für die Hautzellen (vgl. Toxic. in Vitro. Vol. 9, No. 3, Seiten 257 - 266, Elsevier Science Ltd., 1995).

Die Ergebnisse zeigen für beide Cremeformulierungen an den ersten beiden Behandlungstagen eine Erhöhung der Alamarblau-Reduktionsrate und des Glucoseverbrauchs im Vergleich zur unbehandelten Kontrolle (V).

**Tabelle II**

| **Vitalitätsprüfung** | | | |
|---|---|---|---|
| **Rezeptur:** % Farbstoffreduktion pro Std. | **V** **unbehandelt** | **1V** | **3** |
| am 1. Tag | 1,64 | 1,95 | 1,92 |
| am 2. Tag | 1,93 | 2,10 | 2,17 |
| Glucoseverbrauch (in µg/h) | | | |
| am 1. Tag | 59,5 | 68,1 | 83,9 |
| am 2. Tag | 67,4 | 73,4 | 79,6 |

### 3. Prüfung der hautberuhigenden Wirkung am Humanhautmodell (für Creme 3 gemäß Tabelle I)

Auch für diese Prüfung wurde das vorher beschriebene dreidimensionale Hautmodell verwendet.

Zunächst wurde eine Hautreizung durch ein synthetisches Tensidgemisch (Texapon® ASV, 2.5 Gew.-%ig, 1 Stunde) ausgelöst, die eine Ausschüttung der proinflammatorischen Mediatoren Interleukin 1 alpha und Prostaglandin PGE 2 zur Folge hat. Nach dem Abspülen des Tensids wurden Cremeformulierungen auf die Hautstücke aufgetragen. Zum Vergleich wurde eine Cortisonsalbe verwendet, deren hautberuhigende Wirkung bekannt ist.

Für die Messungen wurden jeweils drei Hautkulturen für jede Prüfcreme verwendet und jeweils 3 µl der Creme auf das Filterpapier aufgetragen und dieses mit der gecremten Seite auf die Oberfläche der Hautkultur gelegt und dort 1 Stunde belassen. Nach 24 und nach 48 Stunden (jeweils nach dem Beginn der Hautreizung) wurden die Mengen der in den Hautkulturen freigesetzten Mediatoren bestimmt. Dabei kam die sogenannte ELISA-Technik (enzyme-linked immunosorbant assay) zur Anwendung (vgl. D. Voet. J.G. Voet : Biochemie, VCH Verlagsgesellschaft mbH, Weinheim, 1992, Seite 74 - 75).

In der folgenden Tabelle wird die Menge an Interleukin la und Prostaglandin in Gew.-% bezogen auf die an der unbehandelten Probe 24 Stunden nach der Reizbehandlung gemessenen Menge der Mediatoren (100 %-Wert) angegeben.

**Tabelle III**

| **Hautberuhigende Wirkung** | | | |
|---|---|---|---|
| **Rezeptur** | **unbehandelt** | **Cortison** | **3** |
| Prostaglandin [%] | | | |
| nach 24 Stunden | 100 | 39,8 | 120 |
| nach 48 Stunden | 62 | 25,0 | 26,2 |
| Interleukin 1a [%] | | | |
| nach 24 Stunden | 100 | 69,5 | 66,2 |
| nach 48 Stunden | 26,5 | 25,8 | 14,6 |

## Patentansprüche

1. Kosmetische Zusammensetzungen zur Pflege empfindlicher Haut in Form einer Emulsion oder Creme, **dadurch gekennzeichnet, daß** sie als Öl- oder Fettkomponenten natürliche Öle, Fette oder Wachse oder Ester linearer Fettsäuren mit 8 - 22 C-Atomen, als Emulgatoren Phospholipide, Sterine, Alkyl-(oligo)-glycoside, oder Fettsäureester von Zuckern, Zuckeralkoholen oder Polyglycerin oder Gemischen davon, und als Wirkstoffe Desoxyzucker oder diese enthaltende Naturstoffe enthalten.

2. Kosmetische Zusammensetzungen nach Anspruch 1, **dadurch gekennzeichnet, daß** als Desoxyzucker Fucose, Rhamnose oder ein Gemisch davon in einer Menge von 0,1 - 10 Gew.-% der Zusammensetzung enthalten ist.

3. Kosmetische Zusammensetzung nach einem der Ansprüche 1 oder 2, **dadurch gekennzeichnet, daß** die Öl- oder Fettphase nicht mehr als 5 Gew.-% Kohlenwasserstoffe oder Silikone enthält.

4. Kosmetische Zusammensetzung nach einem der Ansprüche 1 bis 3, **dadurch gekennzeichnet, daß** sie nicht mehr als 1 Gew.-% der Zusammensetzung an Sulfatoder Sulfonattensiden, Ethylenoxid- oder Propylenoxid-Anlagerungsproduke und quartären Ammoniumtensiden enthält.

5. Kosmetische Zusammensetzung nach einem der Ansprüche 1 bis 3, **dadurch gekennzeichnet, daß** sie keine Sulfat- oder Sulfonattenside, Ethylenoxid- oder Propylenoxid-Anlagerungsprodukte oder quartäre Ammoniumtenside enthält.

6. Kosmetische Zusammensetzung nach einem der Ansprüche 1 bis 5, **dadurch gekennzeichnet, daß** als Öl- oder Fettkomponenten Sheabutter (Karite-Fett), Mandelöl, Nachtkerzenöl, ein C₈-C₁₀-Fettsäure-Triglycerid oder ein Gemisch davon in einer Menge von 10 bis 30 Gew.-% der Zusammensetzung enthalten ist.

7. Kosmetische Zusammensetzung nach einem der Ansprüche 1 - 6, **dadurch gekennzeichnet, daß** in der wäßrigen Phase ein natürliches oder synthetisches Hydrocolloid in einer Menge von 0,01 bis 5 Gew.-% der Zusammensetzung enthalten ist.

## Claims

1. Cosmetic compositions for the care of sensitive skin in the form of an emulsion or cream, **characterized in that** they contain natural oils, fats or waxes or esters of linear C₈₋₂₂ fatty acids as oil or fatty components, phospholipids, sterols, alkyl (oligo)glycosides or fatty acid esters of sugars, sugar alcohols or polyglycerols or mixtures thereof as emulsifiers and deoxy sugars or natural substances containing such sugars as active ingredients.

2. Cosmetic compositions as claimed in claim 1, **characterized in that** they contain fucose, rhamnose or a mixture thereof in a quantity of 0.1 to 10% by weight, based on the composition, as the deoxy sugar.

3. Cosmetic compositions as claimed in claim 1 or 2, **characterized in that** the oil or fatty phase contains no more than 5% by weight of hydrocarbons or silicones.

4. Cosmetic compositions as claimed in any of claims 1 to 3, **characterized in that** they contain no more than 1% by weight, based on the composition, of sulfate or sulfonate surfactants, ethylene oxide or propylene oxide adducts and quaternary ammonium surfactants.

5. Cosmetic compositions as claimed in any of claims 1 to 3, **characterized in that** they do not contain any sulfate or sulfonate surfactants, ethylene oxide or propylene oxide addition products or quaternary ammonium surfactants.

6. Cosmetic compositions as claimed in any of claims 1 to 5, **characterized in that** they contain shea butter, almond oil, evening primrose oil, a C₈₋₁₀ fatty acid triglyceride or a mixture thereof in a quantity of 10 to 30% by weight, based on the composition, as the oil or fatty component.

7. Cosmetic compositions as claimed in any of claims 1 to 6, **characterized in that** the aqueous phase contains a natural or synthetic hydrocolloid in a quantity of 0.01 to 5% by weight, based on the composition.

## Revendications

1. Composition cosmétique destinée aux soins de la peau sensible sous forme d'une émulsion ou d'une crème,
**caractérisée en ce qu'**
elles renferme comme composants huileux ou gras, des huiles, des matières grasses ou des cires ou des esters d'acide gras linéaire ayant de 8 à 22 atomes de carbone, comme agents émulsionnants des phospholipides, des stérols, des alkyl(oligo)glycosides, ou des esters d'acide gras de sucres, d'alcools de sucre ou de polyglycérol ou des mélanges de ceux-ci, et comme principes actifs des désoxysucres ou les substances naturelles contenant ceux-ci.

2. Composition cosmétique selon la revendication 1,
**caractérisée en ce que**
comme désoxysucre, le fucose, le rhamnose, ou un mélange de ceux-ci est contenu en une quantité allant de 0,1 à 10 % en poids de la composition.

3. Composition cosmétique selon l'une quelconque des revendications 1 ou 2,
**caractérisée en ce que**
la phase huileuse ou grasse ne contient pas plus de 5% en poids d'hydrocarbures ou de silicones.

4. Composition cosmétique selon l'une quelconque des revendications 1 å 3
**caractérisée en ce qu'**
elle ne renferme pas plus de 1% en poids de la composition en agents tensioactifs du type sulfate ou sulfonate, de produits d'addition de l'oxyde d'éthylène ou de l'oxyde de propylène, et d'agents tensioactifs du type ammonium quaternaire.

5. Composition cosmétique selon l'une quelconque des revendications 1 à 3
**caractérisée en ce qu'**
elle ne contient aucun agent tensioactif du type sulfate ou sulfonate, aucun produit d'addition de l'oxyde d'éthylène ou de l'oxyde de propylène, ou aucun agent tensioactif du type ammonium quaternaire.

6. Composition cosmétique selon l'une quelconque des revendications 1 à 5.
**caractérisée en ce que**
comme composant huileux ou gras, du beurre de karité (Shea Butter), de l'huile d'amande, de l'huile d'onagre, un triglycéride d'acides gras en C₈-C₁₀ ou un mélange de ceux-ci est contenu en quantités allant de 10 à 30 % de la composition.

7. Composition cosmétique selon l'une quelconque des revendications 1 à 6,
**caractérisée en ce que**
dans la phase aqueuse, un hydrocolloïde naturel ou synthétique est contenu en une quantité allant de 0,01 à 5 % en poids de la composition.
